# EUROPÄISCHE PATENTANMELDUNG

(11) **EP 1 714 667 A1**
(43) Veröffentlichungstag der Anmeldung: **25.10.2006**
(21) Anmeldenummer: 06112258.6
(22) Anmeldetag: 05.04.2006
(51) Int. Cl.: A61M 11/00, A61M 5/315, A61M 15/00, B05B 11/00

(54) **Dosiersystem mit einer Tropfer- oder Sprühdüse und zwei angelenkten Schenkeln und Verfahren zu dessen Herstellung**

(30) Priorität: 20.04.2005 CH 7282005
(71) Anmelder: Medisize Schweiz AG, 8309 Nürensdorf (CH)
(72) Erfinder: Fritschi, Isidor, 8540, Andelfingen (CH)
(74) Vertreter: Felber, Josef

(57) **Zusammenfassung**

Der Dosiersystem besteht aus einer Schulter (1) mit Loch (36) und/oder Zylindermündung (32) und zwei an gegenüberliegenden Seiten über Filmscharniere (2,3) daran angelenkten äusseren Schenkeln (4,5) und dazwischen angeordnetem runden Behälter in Form eines Hohlzylinders (6) mit von unten eingeschobenem Kolbensystem (14), wobei die beiden äusseren Schenkel (4,5) über in spitzem Winkel angeformte innere Schenkel (7,8) und über U-förmige Spannbögen (9,10) miteinander verbunden sind, wodurch beim mehrmaligen Zusammendrücken und Loslassen beider Schenkel (4,5) der mit Rastkeilen (15) bestückte Kolben durch die sich nach oben bewegenden Keilschieber (12,13) schrittweise in den Zylinder (6) hineingeschoben und durch die an den Kolbenflügeln (20) vorhandenen Rastelemente (22) nach dem Vorrücken durch am Zylinder vorhandene Flügelschienen (21) fixiert wird, wodurch sich das Zylindervolumen schrittweise verkleinert und die im Zylinder (6) befindliche Lösung schubweise über die Sprühdüse (16) ausgestossen und so appliziert wird.

## Beschreibung

Diese Erfindung betrifft einen Dosiersystem für ein paar wenige Dosen eines Wirkstoffes, insbesondere etwa eines pharmazeutischen Wirkstoffes, sowie ein Verfahren zur Herstellung eines solchen Dosiersystems. Für die Applikation von verschiedenen pharmazeutischen flüssigen Wirkstoffen werden Sprays eingesetzt, das heisst ein Fläschchen oder ein Behälter mit einer aufgeschraubten oder aufgeprellten Spraypumpe oder einem Tropfer. Diese schliesst ein Saugröhrchen ein, das sich in den Behälter oder die Flasche erstreckt sowie einen Pumpenmechanismus und eine Spraydüse. Durch das Betätigen der Pumpe, meist durch Niederdrücken eines Druckknopfes oder einer Drucktaste, wird eine Dosis des Behälterinhaltes durch die Düse gepresst und als Spray versprüht. Typische Anwendungen solcher Sprays sind etwa Hals- und Rachensprays, Nasensprays und auch Ohrensprays. Die Pumpen solcher Sprays sind relativ aufwändig gebaut und teuer in der Herstellung, denn sie bestehen aus mehreren Bestandteilen und diese müssen mit geringen Toleranzen gespritzt werden, um die Dichtigkeit der Pumpe zu gewährleisten und das sichere und dauerhafte Funktionieren für das Sprayen und Tropfen von kleinsten Dosen sicherzustellen. Die Bestandteile müssen aufwändig montiert werden. Es werden jedoch auch Sprays für andere, nicht-medizinische Zwecke eingesetzt, wobei es Sprays mit Spraypumpen wie auch Spraydosen gibt, die mit einem Treibgas gefüllt sind, sodass also zum Sprayen bloss ein Ventil betätigt werden muss. Immer dann, wenn eine Dosis einer Flüssigkeit in feinen Tröpfchen verteilt irgendwo aufgebracht werden soll, eignet sich grundsätzlich ein Spray.

In vielen Fällen wäre es hilfreich und praktisch, für eine gewünschte Anwendung zuerst bloss ein Muster eines Wirkstoffes testen zu können, bevor man einen kompletten Spray mit Spraypumpe kauft. Zum Beispiel möchte man bei einem Farbspray erst testen, ob ein Farbton genau den Wunsch trifft oder zu einer bestehenden Farbe passt, bevor man eine ganze Spraydose mit Spraypumpe einkauft. Das ist besonders dann der Fall, wenn das zu besprayende Objekt nicht zum Farbladen gebracht werden kann, um den Farbton zu ermitteln. Auf dem medizinischen Sektor wäre es praktisch, wenn man zunächst bloss ein kleines Muster eines Wirkstoffes testen könnte, um dessen Wirksamkeit zu prüfen, bevor man ein entsprechendes Behandlungsmittel kauft, etwa einen Nasen-, Hals- oder Rachenspray oder entsprechende Tropfen. Es zeigt sich auch, dass die Kunden bei solchen medizinischen Produkten nicht gerne irgendetwas kaufen, dessen Wirkung für sie nicht gesichert ist, weil solche Behandlungsmittel doch relativ teuer sind, und wenn sie nicht wirken, für den Käufer nutzlos sind.

Für die Anbieter von medizinischen Sprays und Tropfen andererseits wäre es zur Kundengewinnung vorteilhaft, einen Spray oder Tropfer mit einer ganz kleinen Wirkstoffmenge zunächst als Muster abgeben zu können, wobei aber der Wirkstoff trotzdem als Spray oder als Tropfen appliziert werden können sollte. Solche Dosiersysteme für medizinische wie auch nicht-medizinische Zwecke könnten dann von den Anbietern zu bescheidenen Preisen oder gar als Gratismuster abgegeben werden. Damit wären die Kunden bereit, die Wirkstoffe oder Sprayinhalte für ihre Anwendungszwecke zu prüfen und wenn das Resultat sie überzeugt, dann das eigentliche Produkt zu kaufen. In diesem Sinne wäre also ein solches Dosiersystem ein willkommenes Marketinginstrument.

Die Aufgabe der vorliegenden Erfindung besteht deshalb darin, einen Dosiersystem anzugeben, welcher das Applizieren von einer kleinen Anzahl von Tropfen oder Dosen eines Sprays ermöglicht, sowie auch ein Verfahren für die Herstellung eines solchen Dosiersystems anzugeben.

Diese Aufgabe wird gelöst von einem Dosiersystem, bestehend aus einer Sprühdüse oder einem tropfer auf einer Schulter und zwei daran angelenkten äusseren Schenkeln und dazwischen angeordnetem runden Behälter in Form eines Hohlzylinders, wobei die äusseren Schenkel über in spitzem Winkel angeformte innere Schenkel und über U-förmige Spannbögen miteinander verbunden und mit Mitteln zum schrittweisen wiederholten Zusammenpressen versehen sind.

Das Verfahren zum Herstellen eines solchen Dosiersystems zeichnet sich dadurch aus, dass
a) eine Schulter mit einem aufgesetzten, verschlossenen Düsenkörper mit zwei an gegenüberliegenden Seiten über Filmscharniere an der Schulter angelenkten äusseren und inneren Schenkeln aus Kunststoff gespritzt wird, wobei diese über je einen U-förmigen Spannbogen verbunden sind, sowie ein an der Unterseite der Schulter angeformter Zylinder;
b) der Zylinder von unten her durch dessen untere Öffnung befüllt wird;
c) der Zylinder an seinem offenen, unteren Ende mit einem Kolbensystem bestehend aus Kolbenschwanz, Kolbenstift mit darauf aufgesetztem Kolbenkopf sowie Kolbenflügeln, bestückt und somit verschlossen wird.

Das Verfahren zum Herstellen eines solchen Dosiersystems kann sich als Alternative zum oben erklärten Verfahren dadurch auszeichnen, dass
a) eine Schulter mit einem Loch mit zwei an gegenüberliegenden Seiten über Filmscharniere an der Schulter angelenkten äusseren und inneren Schenkeln aus Kunststoff gespritzt wird, wobei diese über je einen U-förmigen Spannbogen verbunden sind, sowie ein an der Unterseite der Schulter angeformter Zylinder;
b) der Zylinder an seinem offenen, unteren Ende mit einem Kolbensystem bestehend aus Kolbenschwanz, Kolbenstift mit darauf aufgesetztem Kolbenkopf sowie zwei Kolbenflügeln, bestückt und somit verschlossen wird;
c) der Zylinder von der Aussenseite der Schulter her durch deren Loch befüllt wird;
d) das Loch in der Schulter auf der Schulteraussenseite mit einer Spraydüse mit aufgesetztem Gabelelement oder einem Tropfer verschlossen wird.

Ein solcher Dosiersystem wird in den Zeichnungen in verschiedenen Ansichten gezeigt und in der nachfolgenden Beschreibung wird sein Aufbau und seine Funktion erklärt. Dabei werden auch die Verfahren zur Herstellung des erfindungsgemässen Dosiersystems beschrieben und erläutert.

Es zeigt:
- Figur 1:: Das Dosiersystem mit eingesetztem Kolbensystem und Spraydüse mit Gabelelement in einer perspektivischen Ansicht von der Seite her gesehen;
- Figur 2:: Das Dosiersystem mit eingesetztem Kolbensystem und Spraydüse mit Gabelelement wie in Figur 1 dargestellt, jedoch in einem Längsschnitt durch seine Mitte gezeigt;
- Figur 3:: Das Dosiersystem mit eingesetztem Kolbensystem und Spraydüse mit abgeschertem Gabelelement in einer perspektivischen Ansicht von der Seite her gesehen;
- Figur 4:: Das Kolbensystem bestehend aus Kolbenschwanz mit Kolbenflügeln und Kolbenstift sowie den Kolbenkopf schematisch von vorne dargestellt;
- Figur 5:: Das Kolbensystem bestehend aus Kolbenschwanz mit Kolbenflügeln wie in Figur 4 dargestellt, jedoch mit aufgesetztem Kolbenkopf schematisch von vorne dargestellt;
- Figur 6:: Das Dosiersystem mit eingesetztem Kolbensystem und Spraydüse mit abgeschertem und umgekehrt wieder aufgesetztem Gabelelement in einer perspektivischen Ansicht von der Seite her gesehen;
- Figur 7:: Einen Längsschnitt durch den Zylinder und eine Düsenolive, aufgesetzt auf die Zylindermündung der Schulter des Mustersprays.

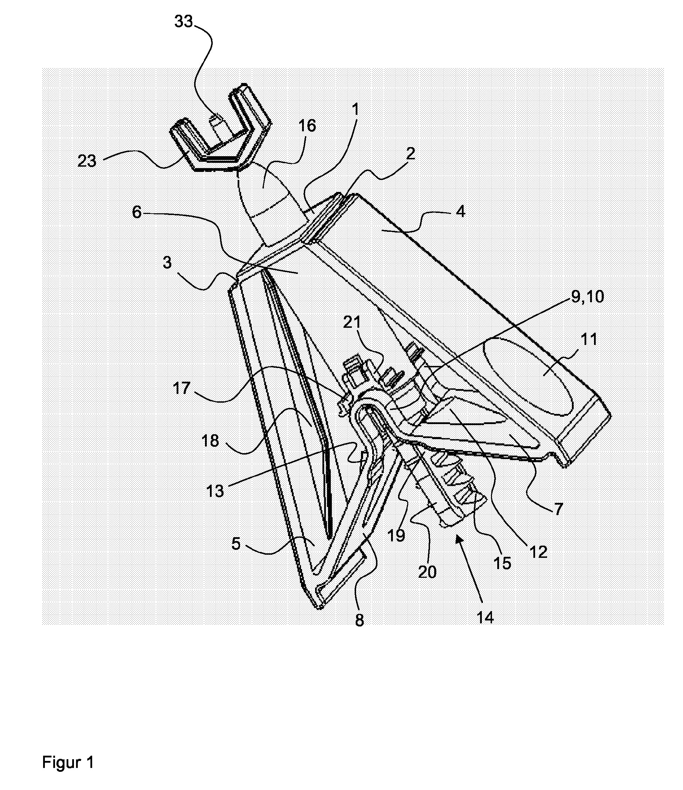

In Figur 1 ist das Dosiersystem mit eingesetztem Kolbensystem 14 und Spraydüse 16 mit Gabelelement 23 in einer perspektivischen Ansicht von der Seite her gesehen, dargestellt. Das Dosiersystem besteht aus einer Schulter 1, die im Zentrum ein hier nicht sichtbares Loch aufweist, sowie aus zwei an gegenüberliegenden Seiten über Filmscharniere 2,3 daran angelenkten äusseren Schenkeln 4,5, sowie einem an der Unterseite um das Loch der Schulter angeformten Hohlzylinder 6. An den äusseren Schenkeln 4,5 sind in spitzem Winkel innere Schenkel 7,8 angeformt, wobei diese durch zwei U-förmige Spannbögen 9,10 derart miteinander verbunden sind, dass der Zylinder 6 zentral zwischen die beiden Spannbögen 9,10 positioniert ist. Die äusseren Schenkel 4,5 zeigen am unteren Ende ovale Einbuchtungen 11 auf, die beim Zusammendrücken der äusseren Schenkel 4,5 mit einer Hand die beteiligten Finger, z.B. Daumen und Zeigefinger fixieren helfen, wodurch das Abrutschen der Finger von den Schenkeln verhindert wird. Die inneren Schenkel 7,8 weisen auf der dem Zylinder zugewandten Seite Keilschieber 12,13 auf, deren Funktion später noch genauer erläutert wird. Es genügt hier zu erwähnen, dass beim Zusammendrücken der beiden äusseren Schenkel 4,5 gegeneinander die Keilschieber 12,13 das Kolbensystem 14 mit seinen Rastkeilen 15 schrittweise in den Zylinder 6 hineinstossen. Die inneren Schenkel 7,8 mit den U-förmigen Spannbögen 9,10 werden dabei nach oben in Richtung Düsenkörper 16 bewegt, wobei diese Bewegung durch vier an der Zylinderwand angebrachte L-förmigen Führungsklötze 17 sowie durch die an der Innenseite der äusseren Schenkel 4,5 mittig in deren Längsrichtung vorhandene dreiecksförmige Schenkelflügel 18 geführt und beschränkt wird, insofern dass die Bewegung nur bis zum Anschlag der Keilschieber 12,13 an die Schenkelflügel 18 möglich ist, wobei das Ausmass der Bewegung, also des vertikalen Kolbenhubes im Zylinder, derart definiert ist, dass das Kolbensystem 14 bei jedem Zusammenpressen der Schenkel 4,5 sich schrittweise um die Länge eines Rastkeils 15 vorwärts bewegt. Die beiden seitlich gegenüberliegend am Kolbenschwanz 19 vorhandenen Kolbenflügel 20 werden durch zwei an der Zylinderwand angespritzte Flügelschienen 21 geführt, wobei die an den äusseren Seiten der Kolbenflügel 20 vorhandenen Rastelemente 22 schrittweise um ein Rastelement nach oben bewegt werden. Dadurch wird das Kolbensystem nach dem Zusammendrücken in dieser neuen Position fixiert und die inneren 7,8 und äusseren Schenkel 4,5 können beim Wegnehmen des Fingerdrucks relaxieren und in die Anfangsposition zurückkehren, ohne dass sich das Kolbensystem im Zylinder zurück bewegt. Vor dem erstmaligen Gebrauch des Dosiersystems, sprich vor dem ersten Zusammenpressen der beiden Schenkel 4,5, muss natürlich das am Düsenkörper 23 angespritzte Gabelelement 24 abgeschert werden. In Figur 1 ist das Dosiersystem mit auf der Schulter 1 aufgesetztem oder aufgespritztem Düsenkörper 16 mit gefülltem Zylinder vor dem ersten Einsatz gezeigt.

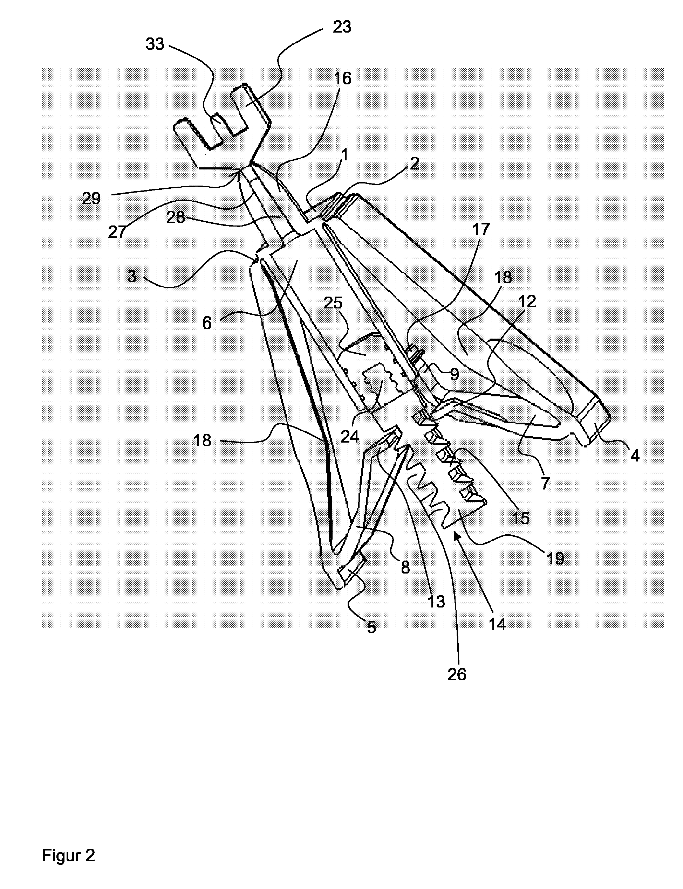

In Figur 2 ist das Dosiersystem mit eingesetztem Kolbensystem 14 und aufgesetztem oder aufgespritztem Düsenkörper 16 mit Gabelelement 23 wie in Figur 1 dargestellt, jedoch in einem Längsschnitt durch seine Mitte gezeigt, mit gefülltem Zylinder vor dem ersten Einsatz. Das Kolbensystem 14 besteht aus Kolbenschwanz 19, Kolbenstift 24 mit Aussengewinde und aufgeschraubtem oder auch im 2-Komponenten-Spritzgiessverfahren hergestellten Kolbenkopf 25, wobei in Ausgangsstellung, also vor dem ersten Gebrauch, sich nur der Kolbenkopf 25 im Zylinder 6 befindet, und die Kolbenflügel 20 in dieser Darstellung nicht zu sehen sind. Vor dem ersten Gebrauch befinden sich die Keilschieber 12,13 der inneren Schenkel 7,8 in der obersten Position des Kolbenschwanzes 19, also in den beiden obersten Kerben 26 des Kolbenschwanzes 19. In der Figur 2 ist der Düsenkörper 16 in Form einer Olive als einfache Variante direkt auf der Schulter 1 aufgespritzt resp. integriert, so dass Düsenbohrung 27 und Düsenkanal 28 identisch sind und an der Düsenmündung 29 ein Gabelelement 23 angeformt ist.

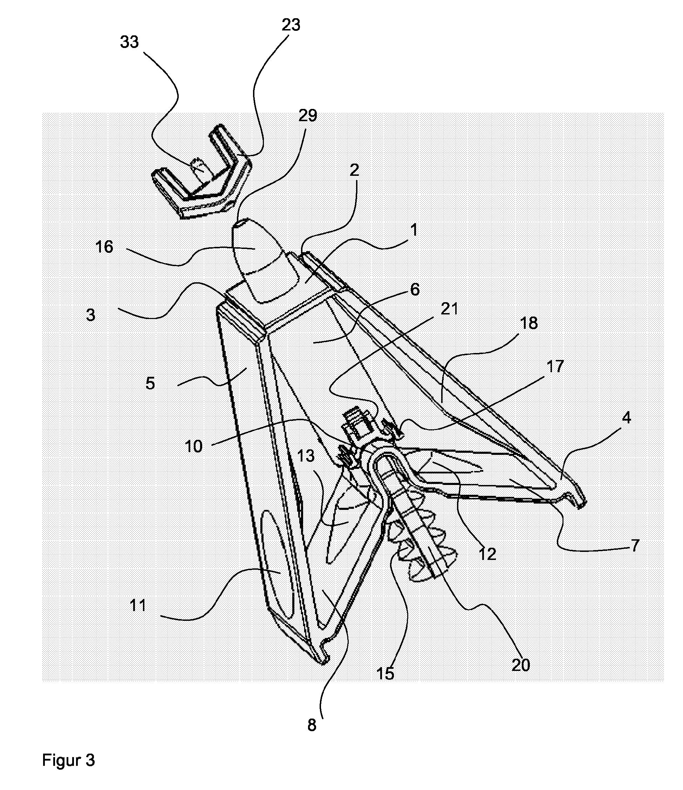

Um dieses Dosiersystem für den ersten Gebrauch bereit zu machen, muss nur das Gabelelement 23 vom Düsenkörper 16 mit einer Drehbewegung abgeschert werden, so dass die Düsenmündung 29 frei wird und der olivenförmige Düsenkörper in das Nasenloch eingeführt werden kann. Die Figur 3 zeigt das Dosiersystem mit eingesetztem Kolbensystem und Spraydüse mit abgeschertem Gabelelement 23 in einer perspektivischen Ansicht von der Seite her gesehen. Ist also das Gabelelement 23 abgeschert und der Düsenkörper 16 in das Nasenloch eingeführt, erfolgt ein elastisches Zusammendrücken der äusseren Schenkel 4,5, wobei die Keilschieber 12,13 das Kolbensystem 14 nach oben bewegen und somit ein genau bestimmtes Volumen der im Zylinder vorhandenen Flüssigkeit durch den dünnen Düsenkanal 28 in die Nase zerstäubt wird. Die beiden Keilschieber 12, 13 werden beim Zusammendrücken der äusseren Schenkel 4,5 in die oberste Kerbe 26 gepresst, wodurch die Bewegung des Kolbensystems 14 in Richtung Düsenkörper 16 ausgelöst wird, so dass auch die inneren Schenkel 7,8 eine aufwärtsgerichtete Bewegung erfahren und die U-förmige Spannbögen 9,10 ebenfalls in Richtung Düsenkörper 16 zwischen den Führungsklötzen 17 aufwärts bewegt werden. Das Ausmass dieser Bewegung wird durch die Schenkelflügel 18 begrenzt, indem die äusseren Schenkel 4, 5 bis zum Anschlag der Schenkelflügel 18 an die Keilschieber 12,13 zusammengepresst werden können, jedoch nicht weiter. Dabei schieben sich die beiden sich gegenüberliegenden, am Kolbenschwanz 19 befindlichen Kolbenflügel 20 um genau ein Rastelement 22 nach oben durch die beiden an der Zylinderwand fixierten Flügelschienen 21 hindurch. Nach applizierter, zerstäubter Lösung eines bestimmten Zylindervolumes in die Nase kann das Dosiersystem relaxieren, d.h. sich in seine ursprüngliche Form zurück bewegen, indem der Druck von den beiden äusseren Schenkeln 4,5 durch Öffnen der Hand resp. der Finger weggenommen wird. Die U-förmigen, unter Druckspannung stehenden Spannbögen 9,10 drücken beim Relaxieren des Systems die inneren Schenkel 7,8 zurück in die ursprüngliche Startposition. Sehr zentral bei diesem Vorgang ist jedoch, dass das Kolbensystem 14 nicht relaxiert, d.h. in seiner nach der ersten Anwendung eingenommenen Position verharrt, indem die Keilschieber 13,14 bei der Relaxation aus der obersten Kerbe 26 über die obersten, tannenbaumförmigen Rastkeile abgleiten, so dass die Keilschieber 13,14 für die nächste Applikation der Lösung in die zweitoberste Kerbe des Kolbenschwanzes 19 einhaken können. Das Kolbensystem 14 wird in seiner Position gehalten, weil sich die beiden Kolbenflügel 20 wegen ihren beim Zusammenpressen durch die Flügelschienen 21 hindurchgeklickten Rastelemente 22 nicht mehr nach unten bewegen können. Das innere Volumen des Zylinders wird also schrittweise, irreversibel bei jeder Kontraktion der äusseren Schenkel 4, 5 verkleinert, wobei jedes Mal eine genau definierte, gleichgrosse Menge Lösung in die Nase appliziert wird. Das Dosiersystem kann so viele Male benutzt werden, wie Anzahl Kerben 26 im Kolbenschwanz 19 vorhanden sind, wobei das Kolbensystem 14 dann vollständig in den Zylinder eingeführt worden ist, so dass der Kolbenkopf 25 den inneren Rand der Schulter 1 berührt und die gesamte Menge Lösung appliziert worden ist. Durch Variation der Länge des Kolbenkopfs 25 kann die Anzahl Stosshübe auch kleiner sein, da bei verlängertem Kolbenkopf 25 bei gleicher Zylindergeometrie und -volumen dieser nach einer kleineren Anzahl Kontraktionen bereits an der inneren Schulter 1 anstösst. Damit kann, je nach Art der zu applizierten Lösung und der gewünschten Wirkung die Anzahl der durchgeführten Kontraktionen und somit des total zur Verfügung stehenden Flüssigkeitsvolumens mittels unterschiedlich langer Zylinderköpfe gesteuert werden, wobei alle anderen Teile des Dosiersystems unverändert bleiben in Gestalt und Geometrie.

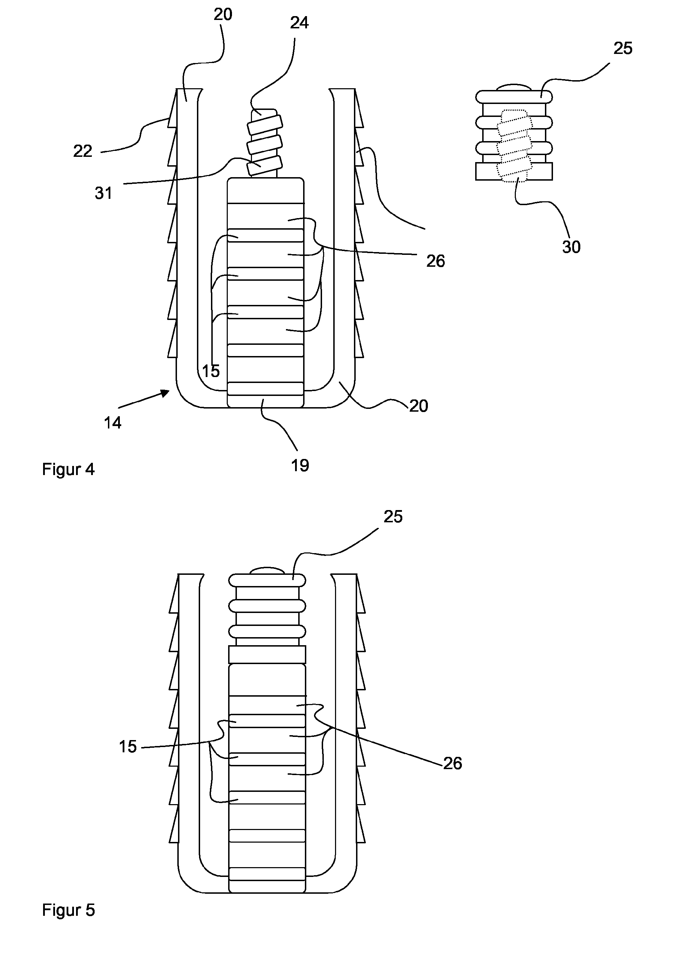

**[0011]** In Figur 4 ist das Kolbensystem 14 bestehend aus Kolbenschwanz 19 mit den Kolbenflügeln 20 und dem Kolbenstift 24 sowie dem Kolbenkopf 25, nicht aufgeschraubt, schematisch von vorne dargestellt. Gut zu erkennen in dieser Ansicht sind die an den äusseren Seiten der beiden Kolbenflügel 20 vorhandenen Rastelemente 22. Diese Rastelemente werden beim schrittweisen Vorrücken des Kolbensystems 14 in den Zylinder durch die beiden an der Zylinderwand befestigten Flügelschienen 21 gepresst, wobei die Rastelemente 22 ähnlich wie Widerhaken ein Zurückgleiten des Kolbensystems 14 verhindern, da diese sich nicht mehr rückwärts durch die Flügelschienen 21 bewegen können. Der kreisförmige Kolbenschwanz 19 ist mit Kerben 26 und Rastkeilen 15 versehen. Vor dem ersten Gebrauch befinden sich die beiden Keilschieber 12,13 in der obersten Kerbe des Kolbenschwanzes 19, wobei beim Zusammendrücken der äusseren Schenkel 4,5 die Keilschieber in die Kerbe 26 einhaken und eine Aufwärtsbewegung ausführen, so dass des Kolbensystem 14 nach oben gedrückt wird. Werden die Schenkel nun wieder losgelassen, also bei Relaxation des Systems, schwenken die Schenkel 4,5,7,8 wieder zurück, ausgelöst durch die U-förmigen Spannbögen 9,10, wobei jedoch das Kolbensystem 14 durch die an den Flügelschienen 21 eingehakten Rastelemente 22 an der Rückwärtsbewegung gehindert wird.

Vor Einfügen des Kolbensystems in den Zylinder wird der Kolbenkopf 25, der, sofern er nicht im 2-Komponenten-Spritzgiessverfahren einteilig hergestellt wird, ein Innengewinde 30 aufweist, auf den Kolbenstift mit Aussengewinde geschraubt. Durch die Variation der Länge des Kolbenkopfes 25 kann das für die Flüssigkeit zur Verfügung stehende Zylindervolumen gesteuert werden. Je nach Bedarf, z.B. bei sehr wertvollen Substanzen ist es also möglich durch Einsatz des entsprechenden Kolbenkopfs 25, dass das Dosiersystem auf z.B. zwei Applikationen limitiert wird. In Figur 5 ist das Kolbensystem 14 wie in Figur 4, jedoch mit aufgesetztem Kolbenkopf schematisch von vorne dargestellt. Das Befüllen des Zylinders 6 geschieht vor dem Einsetzen des Kolbensystems 14 bei verschlossenem Düsenkörper 16 von unten - es ist aber auch möglich, dass das Kolbensystem 14 bereits in den Zylinder eingeführt ist, so dass die Befüllung des Hohlzylinders von oben her erfolgt durch die Zylindermündung 32, wobei dann der Düsenkörper 16 anschliessend aufgesetzt wird.

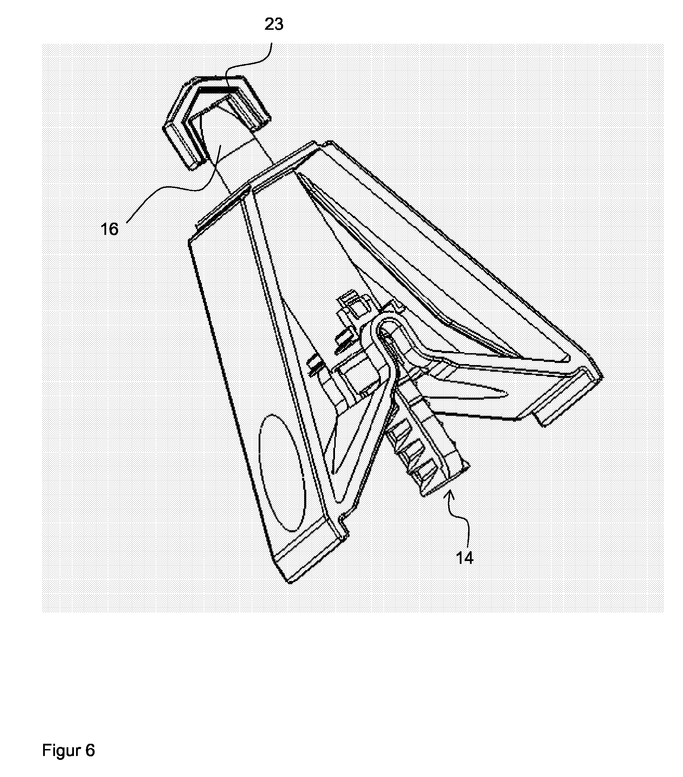

In Figur 6 sieht man das Dosiersystem mit eingesetztem Kolbensystem 14 und Spraydüse 16 mit abgeschertem und umgekehrt wieder aufgesetztem Gabelelement 23 in einer perspektivischen Ansicht von der Seite her gesehen, in Gebrauchslage. Nach dem Abscheren des Gabelelements 23 kann der mittig im Gabelelement 23 vorhandene Zapfen 33 in die Düsenbohrung 34 eingesetzt werden, so dass das Dosiersystem resp. der Hohlzylinder mit der darin befindlichen Flüssigkeit dadurch bis zur nächsten Applikation hermetisch luftdicht geschlossen ist. Bei erneutem Gebrauch kann des Gabelelement 23 durch leichtes Drehen von der Düsenbohrung 34 entfernt und nach Verwendung leicht wieder verschlossen werden.

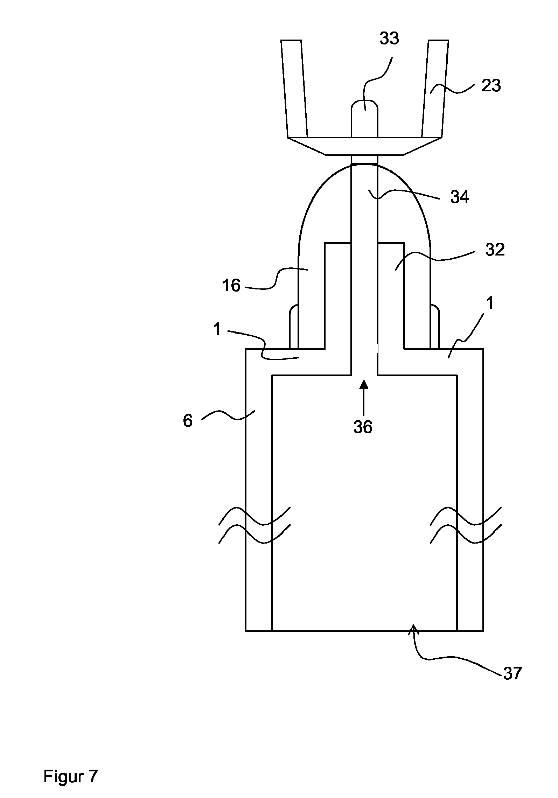

In Figur 7 ist ein Längsschnitt durch den Zylinder 6 und die Düsenolive 16 als Variante mit aufsetzbarem Düsenkörper dargestellt. Die Schulter 1 am oberen Ende des Zylinders 6 verjüngt sich zur Zylindermündung 32, auf die der Düsenkörper 16 in der Form einer halben Olive aufgesetzt ist, wobei die Düsenbohrung 34 sich an der Zylindermündung 32 fortsetzt bis zur Düsenmündung 29, wo die Flüssigkeit exakt dosiert tropft oder unter Druck fein zerstäubt wird. Eine derart geformte Tropfer- oder Sprühdüse eignet sich besonders für einen Nasenspray, weil die olivenförmige Düse in ein Nasenloch geschoben werden kann. Vor dem ersten Gebrauch befindet sich an der Düsenmündung ein aufgespritztes Gabelelement 23, das durch eine Drehbewegung abgeschert werden kann. Alternativ kann die Düsenbohrung 27 auch durch einen Stöpsel 35 verschlossen sein, wobei dieser einzig durch Dünnstellen in der Düsenbohrung 35 gehalten ist und durch Drehen aus der Bohrung durch Reissen der Dünnstellen weggeschert und herausgedreht werden kann.

## Patentansprüche

1. Dosiersystem, bestehend aus einer Tropfer- oder Sprühdüse (16) auf einer Schulter (1) und zwei daran angelenkten äusseren Schenkeln (4,5) und dazwischen angeordnetem runden Behälter in Form eines Hohlzylinders (6), wobei die äussern Schenkel (4,5) über in spitzem Winkel angeformte innere Schenkel (7,8) und über U-förmige Spannbögen (9, 10) miteinander verbunden und mit Mitteln (12,13,15,17,18,20-22) zum schrittweisen wiederholten Zusammenpressen versehen sind.

2. Dosiersystem nach Anspruch 1, bestehend aus einer Schulter (1) mit Loch (36) und zwei an gegenüberliegenden Seiten über Filmscharniere (2,3) daran angelenkten äussere Schenkeln (4,5), sowie einer an der Innenseite um das Loch (36) der Schulter (1) angeformter Hohlzylinder (6) und auf der gegenüberliegenden Seite am Loch (36) angesetzte Spraydüse (16), wobei die äusseren Schenkel (4,5) über in spitzem Winkel angeformte innere Schenkel (7,8) und über U-förmige Spannbögen (9,10) miteinander verbunden zueinander hin schwenkbar sind.

3. Dosiersystem nach Anspruch 1, bestehend aus einer Schulter (1) mit Loch (36) und Zylindermündung (32) sowie zwei an gegenüberliegenden Seiten über Filmscharniere (2,3) daran angelenkten äussere Schenkeln (4,5), sowie einer an der Innenseite um das Loch (36) der Schulter (1) angeformter Hohlzylinder (6) und auf der gegenüberliegenden Seite auf die Zylindermündung (32) aufgesetzter Düsenkörper (16), wobei die äusseren Schenkel (4, 5) über in spitzem Winkel angeformte innere Schenkel (7, 8) und über U-förmige Spannbögen (9, 10) miteinander verbunden zueinander hin schwenkbar sind.

4. Dosiersystem nach einem der vorangehenden Ansprüche, ***dadurch gekennzeichnet,* dass** er eine an der Innenseite um das Loch (36) der Schulter (1) angeformter Hohlzylinder (6) und ein an der unteren Zylinderöffnung (37) einschiebbares Kolbensystem (14) aufweist, wobei die äusseren Schenkel (4, 5) zueinander hin schwenkbar sind und die inneren Schenkel (7, 8) mit Keilschieber (12, 13) versehen sind, wodurch das Kolbensystem (14) schrittweise nach oben geschoben wird.

5. Dosiersystem nach einem der vorangehenden Ansprüche, ***dadurch gekennzeichnet,* dass** das in den Zylinder (6) einschiebbare Kolbensystem (14) aus Kolbenflügel (20) mit daran an der Aussenseite vorhandenen Rastelementen (22), aus einem Kolbenschwanz (19) mit integrierten Rastkeilen (15) und Kerben (26), einem Kolbenstift (24) mit Aussengewinde (31) und einem darauf aufschraubbaren Kolbenkopf (25) mit Innengewinde (30) besteht.

6. Dosiersystem nach einem der vorangehenden Ansprüche, ***dadurch gekennzeichnet,* dass** beidseits an der Zylinderaussenwand Flügelschienen (21) angeformt sind, durch die beim Zusammenschwenken der Schenkel (4, 5, 7, 8) durch den Druck der Rastkeile (15) in die Kerben (26) sich die Kolbenflügel (20) mit ihren Rastelementen (22) hindurchschieben und irreversibel einhaken, sowie dass angrenzend an die Flügelschienen (21) an der Zylinderaussenwand Führungsklötze (17) angeformt sind, durch die beim Zusammenschwenken der Schenkel (4, 5, 7, 8) sich in Richtung Düsenkörper bewegende U-förmige Spannbögen (9, 10) hindurchschieben.

7. Dosiersystem nach einem der vorangehenden Ansprüche, ***dadurch gekennzeichnet,* dass** die äusseren Schenkel (4,5) an ihrer Innenseite Einbuchtungen (11) für den sicheren Griff der Finger aufweisen und dass diese Schenkel auf der Innenseite längs mittig mit Schenkelflügeln (18) ausgerüstet sind, die beim Zusammendrücken der Schenkel (4,5,7,8) auf die Keilschiebern (12, 13) treffen und so diese Bewegung limitieren.

8. Dosiersystem nach einem der vorangehenden Ansprüche, ***dadurch gekennzeichnet,* dass** die Spraydüse für einen Nasenspray einen Düsenkörper (16) in Form einer Olivenhälfte aufweist.

9. Dosiersystem nach einem der vorangehenden Ansprüche, ***dadurch gekennzeichnet,* dass** die Spraydüse ein aufgespritztes Gabelelement (23) aufweist, das mittig einen Zapfen (33) trägt, der nach dem Abscheren des Gabelelements (23) zum Verschliessen der Düsenbohrung (34) auf den Düsenträger (16) für den mehrmaligen Gebrauch aufgesetzt wird.

10. Dosiersystem nach einem der vorangehenden Ansprüche, ***dadurch* gekennzeichnet, dass** die Spraydüse ein Gabelelement (23) mit Zapfen (33) und gegenüberliegendem Stöpsel (35) aufweist, der durch Dünnstellen bis zum ersten Gebrauch in der Düsenbohrung (27) gehalten ist und durch Drehen aus der Bohrung durch Reissen der Dünnstellen weggeschert und herausgedreht werden kann, wobei mittels des Zapfens (33) die Düsenbohrung (34) des Düsenträgers (16) für den mehrmaligen Gebrauch geöffnet und geschlossen wird.

11. Verfahren zum Herstellen eines Dosiersystems nach Anspruch 1, ***dadurch gekennzeichnet,* dass**
a) eine Schulter (1) mit einem aufgesetzten, verschlossenen Düsenkörper (16) mit zwei an gegenüberliegenden Seiten über Filmscharniere (2,3) an der Schulter angelenkten äusseren und inneren Schenkeln (4,5,7,8) aus Kunststoff gespritzt wird, wobei diese über U-förmigen Spannbögen (9,10) verbunden sind, sowie ein an der Unterseite der Schulter angeformter Hohlzylinder (6);
b) der Zylinder (6) von unten her durch dessen untere Öffnung (37) befüllt wird;
c) der Zylinder (6) an seinem offenen, unteren Ende (37) mit einem Kolbensystem (14) bestehend aus Kolbenschwanz (19), Kolbenstift (24) mit darauf aufgesetztem Kolbenkopf (25) sowie Kolbenflügeln (20), bestückt und somit verschlossen wird.

12. Verfahren zum Herstellen eines Dosiersystems nach Anspruch 1, ***dadurch gekennzeichnet,* dass**
a) eine Schulter (1) mit einem Loch (36) mit Zylindermündung (32) mit zwei an gegenüberliegenden Seiten über Filmscharniere (2,3) an der Schulter (1) angelenkten äusseren und inneren Schenkeln (4,5,7,8) aus Kunststoff gespritzt wird, wobei diese über je einen U-förmigen Spannbogen (9,10) verbunden sind, sowie ein an der Unterseite der Schulter (1) angeformter Hohlzylinder (6);
b) der Zylinder (6) an seinem offenen, unteren Ende (37) mit einem Kolbensystem (14) bestehend aus Kolbenschwanz (19), Kolbenstift (24) mit darauf aufgesetztem Kolbenkopf (25) sowie Kolbenflügeln (20), bestückt und somit verschlossen wird;
c) der Zylinder (6) von der Aussenseite der Schulter her durch deren Loch (36) mit Zylindermündung (32) befüllt wird;
d) das Loch (36) mit Zylindermündung (32) in der Schulter (1) auf der Schulteraussenseite mit einer Spraydüse (16) mit aufgespritztem Gabelelement (23) verschlossen wird.
